# FASCICULE DE BREVET EUROPEEN

(11) **EP 3 703 718 B1**
(45) Date de publication et mention de la délivrance du brevet: **19.01.2022**
(21) Numéro de dépôt: 18811060.5
(22) Date de dépôt: 30.10.2018
(51) Int. Cl.: A61K 31/7048, A61K 39/39, C07K 16/28, A61K 39/00, A61K 9/127, A61P 35/00, A61K 45/06, A61K 35/74

(54) **FORMULATION LIPOSOMALE DE LIPOPOLYSACCHARIDE BACTÉRIEN COMBINÉE À UN AGENT CYTOTOXIQUE, ET SON UTILISATION EN THERAPIE ANTI-TUMORALE**
LIPOSOMALE FORMULIERUNG EINES BAKTERIELLEN LIPOPOLYSACCHARIDS KOMBINIERT MIT EINEM ZYTOTOXISCHEN WIRKSTOFF, UND VERWENDUNG ALS ANTITUMORMITTEL
LIPOSOMAL FORMULATION OF BACTERIAL LIPOPOLYSACCHARIDE COMBINED WITH A CYTOTOXIC AGENT, AND ITS USE AS ANTI-TUMOR AGENT

(30) Priorité: 30.10.2017 FR 1760209
(43) Date de publication de la demande: 09.09.2020
(73) Titulaire: Institut National de la Santé et de la Recherche Médicale (INSERM), 75013 Paris (FR); Centre National de la Recherche Scientifique - CNRS, 75016 Paris (FR); Université Claude Bernard Lyon 1, 69100 Villeurbanne (FR); Hospices Civils De Lyon, 69002 Lyon (FR); Centre Léon Bérard, 69008 Lyon (FR)
(72) Inventeur: DUMONTET, Charles, 69200 Venissieux (FR); CHETTAB, Abdelkamel, 69008 Lyon (FR)
(74) Mandataire: Regimbeau
(86) Numéro de dépôt international: PCT/FR2018/052695
(87) Numéro de publication internationale: WO 2019/086806

(56) Documents cités:
- WO-A1-98/51217
- WO-A1-2013/129936
- WO-A1-2017/035009
- DUMONT S ET AL: "ANTITUMORAL PROPERTIES AND REDUCED TOXICITY OF LPS TARGETED TO MACROPHAGES VIA NORMAL OR MANNOSYLATED LIPOSOMES", ANTICANCER RESEARCH, vol. 10, no. 1, 1990, pages 155-160, XP009506044, ISSN: 0250-7005
- SHAHAR EHUD ET AL: "Targeted microbeads for attraction and induction of specific innate immune response in the tumor microenvironment.", VACCINE 21 OCT 2010, vol. 28, no. 45, 21 octobre 2010 (2010-10-21), pages 7279-7287, XP002782021, ISSN: 1873-2518
- ALVING CARL R ET AL: "Lipid A and liposomes containing lipid A as antigens and adjuvants", VACCINE, vol. 26, no. 24, juin 2008 (2008-06), pages 3036-3045, XP022703341, ISSN: 0264-410X
- JUNG SARA ET AL: "Preclinical progress and first translational steps for a liposomal chemotherapy protocol against adrenocortical carcinoma.", ENDOCRINE-RELATED CANCER 10 2016, vol. 23, no. 10, octobre 2016 (2016-10), pages 825-837, XP002788424, ISSN: 1479-6821
- WATSON DOUGLAS S ET AL: "All-trans retinoic acid potentiates the antibody response in mice to a lipopeptide antigen adjuvanted with liposomal lipid A.", IMMUNOLOGY AND CELL BIOLOGY, vol. 87, no. 8, novembre 2009 (2009-11), pages 630-633, XP002788425, ISSN: 1440-1711
- MERAZ ISMAIL M ET AL: "Multivalent presentation of MPL by porous silicon microparticles favors T helper 1 polarization enhancing the anti-tumor efficacy of doxorubicin nanoliposomes.", PLOS ONE 2014, vol. 9, no. 4, 2014, page e94703, XP002788426, ISSN: 1932-6203

## Description

### DOMAINE DE L'INVENTION

La présente invention se rapporte au domaine pharmaceutique, et plus particulièrement au domaine de l'oncologie, notamment au domaine du traitement des cancers. Plus particulièrement, la présente invention concerne une composition pharmaceutique destinée à traiter une tumeur, cette composition étant utilisée en tant que telle ou en tant que composition adjuvante d'un autre composé thérapeutique. La présente invention est également relative à un produit de combinaison pharmaceutique.

### ETAT DE LA TECHNIQUE

Le cancer est une maladie caractérisée par la multiplication incontrôlée de cellules au sein d'un organisme, cette multiplication étant liée à des mutations génétiques touchant l'ADN de ces cellules. Ces mutations apparaissent de façon spontanée ou induite, suite à l'exposition à des agents mutagènes, ou encore ont été transmises de manière héréditaire.

Des cellules cancéreuses de diverses origines, ayant une prolifération cellulaire excessive, donnent naissance à une « tumeur » c'est à dire à une masse tissulaire ayant tendance à persister et à croître au sein du tissu d'origine, et éventuellement à se disséminer dans d'autres tissus.

Dans le cas des cellules cancéreuses circulantes, notamment sanguines, ces cellules sont caractérisées par une capacité de croissance et de division anarchique, non contrôlée.

Aujourd'hui, de nombreuses thérapies anti-tumorales existent. De manière non exhaustive, les traitements disponibles comprennent l'ablation chirurgicale de la tumeur, la chimiothérapie (administration de médicaments cytotoxiques destinés à détruire les cellules cancéreuses), la radiothérapie (irradiation de la tumeur), les traitements hormonaux, les traitements anti-angiogénique et l'immunothérapie.

L'immunothérapie est un traitement qui consiste à administrer des substances biologiques, habituellement produites par le système immunitaire, afin de renforcer et/ou de stimuler les défenses immunitaires d'un organisme. Il a en effet été observé que, lors de l'apparition d'une tumeur, l'organisme affecté génère de lui-même une réponse immunitaire contre cette tumeur, les cellules tumorales étant reconnues en tant que telles par le système immunitaire. Cette réponse immunitaire est toutefois généralement insuffisante pour faire disparaître ladite tumeur. Le but de l'immunothérapie est donc de soutenir et/ou de se substituer à cette réponse biologique insuffisante.

Les substances biologiques utilisées en immunothérapie sont par exemple des anticorps, notamment des anticorps monoclonaux, des cytokines, des interleukines, des interférons et d'une manière générale tout composé immunostimulant.

Pour chaque type de tumeur, les cliniciens décident du ou des traitements à appliquer en fonction des protocoles médicaux préalablement validés, et des spécificités de chaque patient. Les traitements actuellement disponibles peuvent encore être améliorés dans la mesure où aucun ne présente une efficacité complète, ni n'est adapté à tous les types de patients.

En immunothérapie, des études ont été réalisées afin de potentialiser les effets bénéfiques de l'administration d'un anticorps thérapeutique. Par exemple, la demande de brevet WO 2013/129936 décrit l'utilisation thérapeutique d'une combinaison constituée d'un anticorps et d'un immuno-modulateur encapsulé dans un matériau particulaire ou vésiculaire. L'immuno-modulateur encapsulé est notamment une cytokine enveloppée dans un liposome, dont la co-administration stimule les effets bénéfiques de l'anticorps.

### Les liposomes

Un liposome est une vésicule artificielle formée par des bicouches lipidiques concentriques, emprisonnant entre elles des molécules. Les liposomes sont le plus couramment composés de phospholipides, d'un seul ou de plusieurs types. Ces phospholipides s'organisent dans un état thermodynamiquement stable tel que les têtes polaires se regroupent entre elles et permettent l'établissement d'une bicouche. Les liposomes sont des structures de taille nanométrique.

Les liposomes peuvent retenir plusieurs types de composés qu'ils soient hydrosolubles (encapsulés dans la phase aqueuse) ou liposolubles ou amphiphiles (empaquetés dans la bicouche lipidique).

L'utilisation des liposomes dans l'industrie pharmaceutique, comme vecteur de diverses molécules d'intérêt biologique, a fait l'objet de nombreux travaux et plusieurs médicaments de ce type sont actuellement approuvés en vue d'une administration par voie intraveineuse.

En effet, l'encapsulation de substances actives dans des liposomes permet d'assurer la protection desdites substances. Elle permet aussi de limiter l'action toxique des substances, et de réguler leur vitesse de libération. De plus, les liposomes permettent le passage de substances hydrosolubles à travers la membrane hydrophobe des cellules.

Les liposomes sont de plus en plus utilisés en thérapie comme vecteurs de médicaments. Leur utilisation première est le ciblage de principes actifs.

### Le lipopolysaccharide bactérien (LPS)

Le lipopolysaccharide bactérien (LPS) est le principal composant de la membrane externe des bactéries de type Gram négatif. Le LPS possède de fortes capacités immunostimulantes : sa présence dans un organisme génère la stimulation de tout le système immunitaire, notamment via la sécrétion de cytokines pro-inflammatoires, en vue de répondre à la contamination bactérienne. En raison de cette réaction immunitaire forte, l'injection de LPS dans sa forme soluble peut être toxique, voire létale à forte dose, pour un organisme de mammifère. Le LPS induit chez l'homme des réactions telles qu'une hyperthermie, une agrégation des hématies, et un choc septique.

La molécule de LPS est composée de trois entités : le lipide A, le noyau et l'antigène O. Le lipide A qui représente la partie la plus toxique du LPS est très conservé, le noyau est très peu variable alors que l'antigène O est spécifique de l'espèce bactérienne de provenance du LPS.

Le mode d'action et les voies de signalisation du LPS sont bien connus. Ses cellules cibles sont principalement les macrophages, les monocytes, les granulocytes et les cellules épithéliales. Une fois libéré dans l'organisme, le LPS se fixe sur la protéine LBP (LPS Binding Protein) synthétisée par le foie, qui permet sa présentation et fixation au récepteur membranaire CD14 des monocytes. Le LPS s'associe alors également avec un corécepteur membranaire MD2 et avec le récepteur TLR 4 sous sa forme homodimérisée. La formation de ce complexe membranaire induit dans la cellule l'activation de la voie MAP-Kinase et la sécrétion de cytokines pro-inflammatoires.

En recherche fondamentale, le LPS est utilisé *in vitro* et *in vivo,* pour induire une inflammation liée à une forte sécrétion de cytokines pro-inflammatoires par des cellules de type monocytes ou macrophages.

En thérapie et surtout en prophylaxie, le LPS ou son entité 'lipide A' est utilisé en tant qu'adjuvant vaccinal, pour stimuler une réponse immunitaire ciblée contre un antigène co-administré avec ledit LPS.

### Formulation liposomale de LPS

Il a été montré que l'encapsulation de LPS dans des liposomes minimise ces effets pro-inflammatoires à la fois *in vitro* et *in vivo* (Bakouche *et al.,* 1987 ; Dijkstra *et al.*, 1989).

En thérapie anti-tumorale, le LPS a été utilisé en tant qu'adjuvant, pour stimuler la réponse immunitaire à un antigène spécifique : Neidhart et al. (Vaccine, 2004) ont décrit une méthode de traitement destinée aux patients atteints de cancer colorectal, comprenant l'administration d'un vaccin thérapeutique constitué d'une formulation liposomale comprenant à la fois une protéine KSA recombinante et du monophosphoryl lipide A (constituant actif du LPS) ; la majorité des patients traités avec ce vaccin thérapeutique ont développé une immunité spécifique contre la protéine KSA.

Une formulation liposomale de LPS a donc déjà été utilisée dans le cadre de la préparation de compositions vaccinales anti-tumorales, le LPS étant utilisé pour ses propriétés d'adjuvant. Sa co-formulation avec un antigène permet d'optimiser la réponse immunitaire spécifique contre cet antigène.

Toutefois, à ce jour, il n'avait jamais été envisagé d'utiliser du LPS en tant que seul principe actif, dans une formulation liposomale, pour un usage thérapeutique.

Par ailleurs, l'association d'une formulation liposomale contenant exclusivement du LPS avec au moins un composé cytotoxique n'avait jamais été envisagée, de même que l'utilisation de ladite association pour traiter des tumeurs.

### EXPOSE DE L'INVENTION

L'encapsulation du LPS dans des liposomes permet de minimiser ses effets toxiques et ainsi d'utiliser cette formulation liposomale en thérapie, notamment dans le cadre d'un traitement thérapeutique anti-tumoral par voie systémique.

La présente invention concerne un produit de combinaison pharmaceutique comprenant :
- une formulation liposomale contenant exclusivement un LPS; et
- au moins un composé cytotoxique, comme par exemple un anticorps thérapeutique ou un composé de chimiothérapie.

L'invention concerne également ledit produit de combinaison thérapeutique pour son utilisation simultanée, séparée ou séquentielle en thérapie anti-tumorale.

La présente invention concerne également une formulation liposomale contenant exclusivement un lipopolysaccharide bactérien (LPS) pour son utilisation thérapeutique anti-tumorale.

De manière plus générale, une formulation liposomale contenant exclusivement un lipopolysaccharide bactérien (LPS) pour son utilisation en tant que médicament est décrite.

### DESCRIPTION DES FIGURES

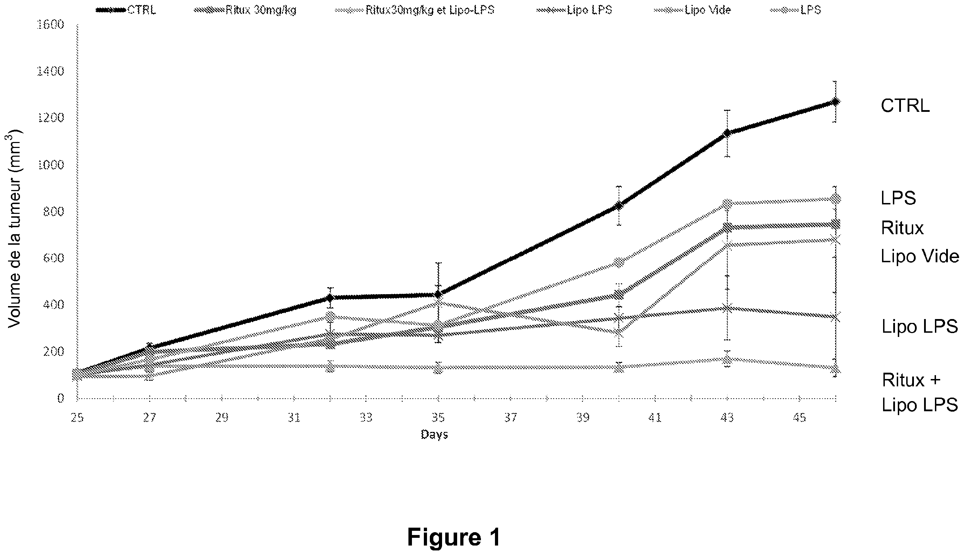

**Figure 1****.** Suivi des volumes tumoraux de souris porteuses de xénogreffes de lymphome RL (cellules de lymphome B humain CD20+ de référence ATCC CRL-2261) non traitées (contrôles) ou traitées avec du LPS non encapsulé, du rituximab, des liposomes vides, une formulation liposomale de LPS et une combinaison de rituximab + formulation liposomale de LPS.

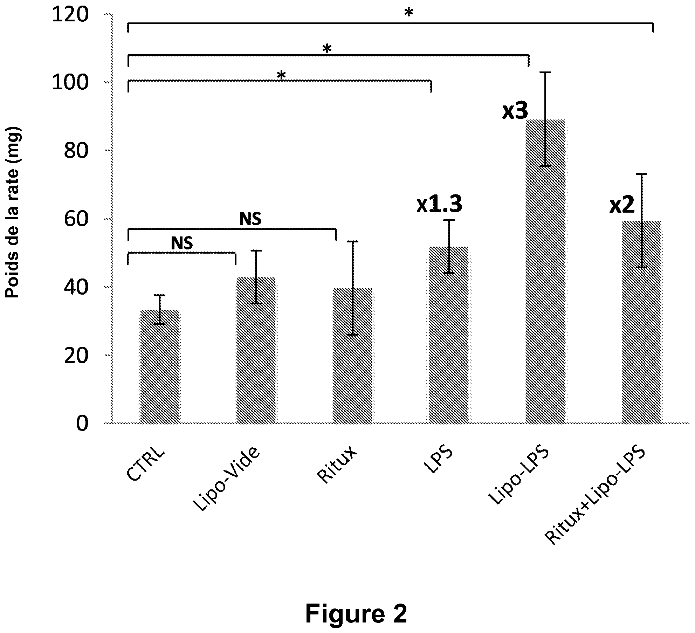

**Figure 2****.** Poids des rates de souris traitées : souris contrôles (CTRL), traitées avec des liposomes vides (Lipo-Vide), traitées avec l'anticorps Rituximab (Ritux), traitées avec du LPS non encapsulé (LPS), traitées avec une formulation liposomale de LPS (Lipo-LPS), et traitées avec une combinaison d'anticorps Rituximab et une formulation liposomale de LPS (Ritux + Lipo-LPS).

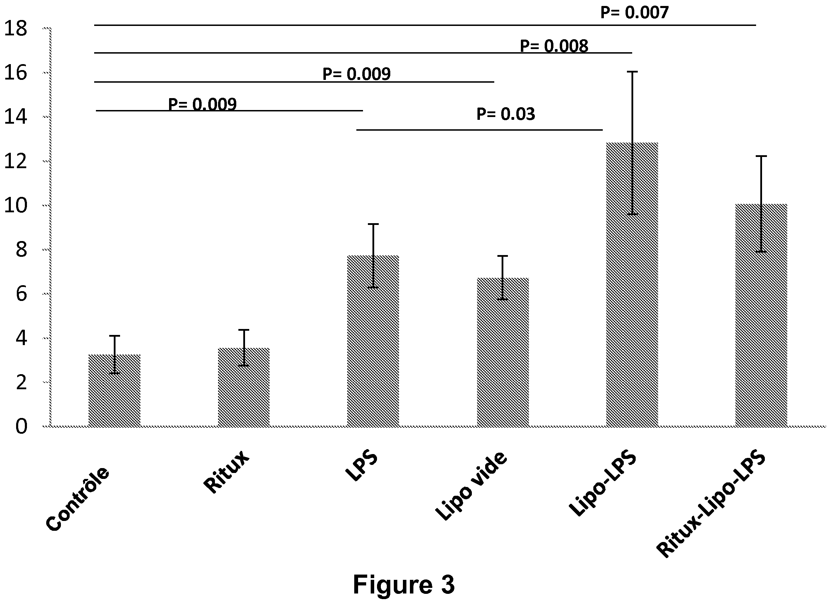

**Figure 3****.** Quantité de cellules NK dans des rates de souris traitées : souris contrôles (contrôle), traitées avec l'anticorps Rituximab (Ritux), traitées avec du LPS non encapsulé (LPS), traitées avec des liposomes vides (Lipo-Vide), traitées avec une formulation liposomale de LPS (Lipo-LPS), et traitées avec une combinaison d'anticorps Rituximab et une formulation liposomale de LPS (Ritux + Lipo-LPS).

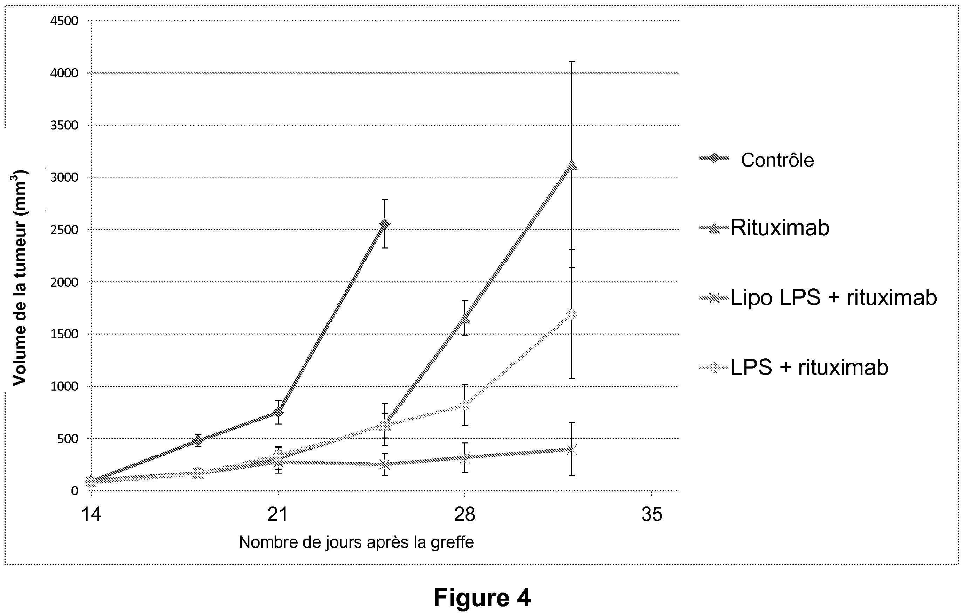

**Figure 4****.** Suivi des volumes tumoraux de souris porteuses de xénogreffes de lymphome RL traitées avec (i) du Rituximab, (ii) une combinaison de Rituximab avec une formulation liposomale de LPS, ou (iii) une combinaison de Rituximab avec du LPS non encapsulé.

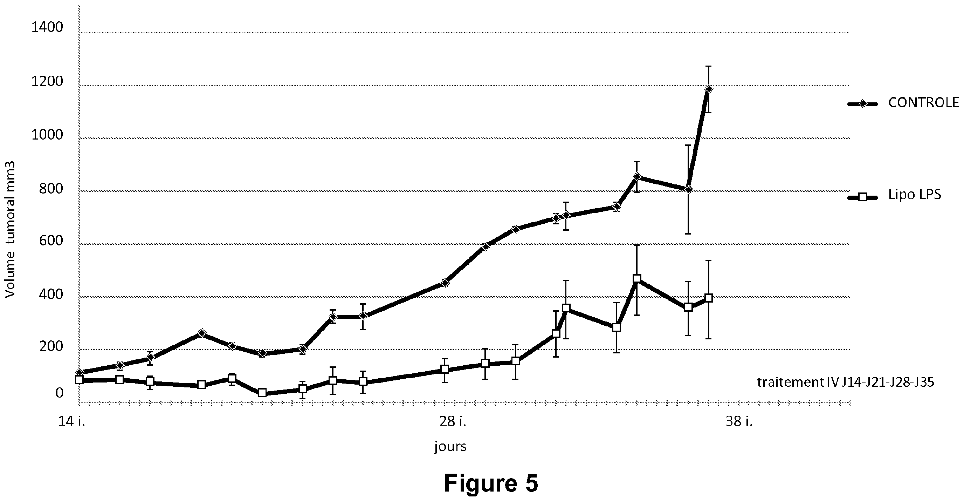

**Figure 5****.** Suivi des volumes tumoraux de souris scid porteuses de xénogreffes de cellules cancéreuses MDA-MB-231, traitées avec de la formulation liposomale de LPS (Lipo LPS, courbe en gris) ou non traitées (CONTROL).

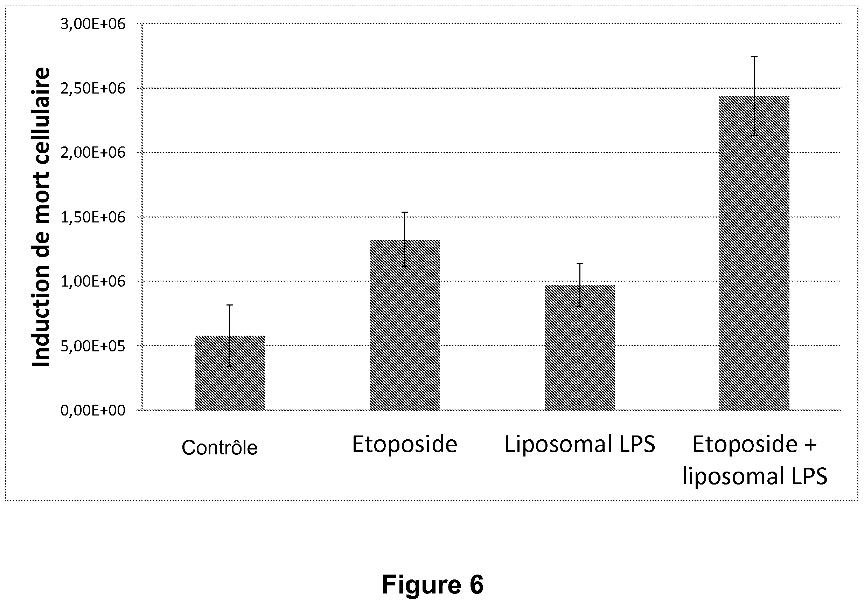

**Figure 6****.** Cellules d'ostéosarcome de rat cultivées *in vitro,* incubées avec :
   - Des macrophages stimulés pendant 16h avec 100 ng/ml de Lipo-LPS WO (LPS-Biosciences), ou
   - 5 µM d'etoposide, ou
   - Une combinaison des deux.

Les cellules contrôles ont été exposées au seul véhicule, ne contenant ni macrophages ni étoposide.

Le taux d'apoptose des cellules cancéreuses a été suivi par un marquage avec le composé IncuCyte^{®} Caspase-3/7.

### DESCRIPTION DETAILLEE DE L'INVENTION

La présente invention se fonde sur l'identification de nouvelles propriétés immuno-stimulatrices du lipopolysaccharide bactérien (LPS). En raison de sa toxicité, ce composé immunostimulant ne peut être utilisé en tant que tel. Le LPS a déja été utilisé en tant qu'adjuvant dans des vaccins, sous une formulation liposomale contenant un antigène et du LPS.

Les inventeurs ont mis en évidence le fait qu'une formulation liposomale de LPS, ne contenant pas d'autres composés et notamment aucun antigène, peut être utilisée en tant que médicament, et plus particulièrement que cette formulation présente des effets bénéfiques thérapeutiques dans le cadre d'un traitement anti-tumeurs.

Selon un premier aspect, l'invention concerne une formulation liposomale contenant exclusivement un lipopolysaccharide bactérien (LPS) pour son utilisation en tant que médicament.

Selon un second aspect, l'invention concerne une formulation liposomale contenant exclusivement un lipopolysaccharide bactérien (LPS) pour son utilisation thérapeutique anti-tumorale.

Autrement dit, la présente invention concerne une formulation pharmaceutique consistant en un LPS bactérien encapsulé dans des liposomes, pour son utilisation en thérapie, notamment pour son utilisation thérapeutique anti-tumorale.

Selon un troisième aspect, l'invention concerne un produit de combinaison pharmaceutique comprenant :
- Une formulation liposomale contenant exclusivement un LPS, et
- Au moins un composé cytotoxique.

Selon un quatrième aspect, l'invention concerne ledit produit de combinaison thérapeutique, pour son utilisation thérapeutique anti-tumorale.

### Définitions

Au sens de l'invention, on entend par «cancer» une pathologie caractérisée par la présence dans un organisme de cellules cancéreuses, formées par transformation de cellules initialement normales de l'organisme atteint par cette pathologie. Un organisme vivant présentant de telles cellules cancéreuses est diagnostiqué comme étant atteint d'un cancer. Il existe près de 200 types de cancers différents, en fonction du tissu où se développe la première tumeur dite tumeur primaire.

Au sens de l'invention, on entend par « tumeur » une masse tissulaire issue d'une prolifération cellulaire excessive de cellules cancéreuses, cette masse tissulaire ayant tendance à persister et à croître de façon non-régulée et autonome vis-à-vis de l'organisme.

La présente invention concerne tous les types de tumeurs, mais plus particulièrement les tumeurs malignes. Les tumeurs malignes ont habituellement une croissance rapide, et ont tendance à récidiver après éradication locale. Les tumeurs malignes sont mal limitées, non encapsulées, et leurs contours sont irréguliers.

La présente invention concerne le traitement des tumeurs primaires et des tumeurs secondaires, issues de la dissémination métastatique d'une tumeur primaire.

Les tumeurs sont généralement classifiées en fonction de leur tissu d'origine : on distingue par exemple les tumeurs de la peau, des os, ou des cellules sanguines.

Deux grandes catégories de tumeurs ont été définies :
- Les tumeurs dites « solides » se développent dans des tissus tels que la peau, les muqueuses, les os et les organes. Ce sont les tumeurs les plus fréquentes : elles représentent 90% des cancers humains.

Parmi les tumeurs solides, on distingue les carcinomes, issus de cellules épithéliales (peau, muqueuses, glandes) ; et les sarcomes, issus de cellules des tissus conjonctifs.
- Les tumeurs dites « liquides » sont issues de cellules sanguines et ne sont pas à proprement parler des excroissances tissulaires, mais sont caractérisées par la présence de cellules sanguines cancéreuses ayant une capacité de croissance et de division anarchique, non contrôlée.

Parmi les tumeurs liquides, on distingue les leucémies (cancers du sang et de la moelle osseuse), caractérisées par la multiplication anarchique de cellules précurseurs des globules blancs dans la moelle osseuse; et les lymphomes (cancers du système lymphatique) qui affectent les lymphocytes.

Selon un aspect particulier de l'invention, la tumeur traitée est une tumeur liquide ou une tumeur solide.

Selon un autre aspect particulier de l'invention, la tumeur traitée est choisie parmi le groupe consistant en : une tumeur de sein, de poumon, de peau (mélanome), de sang (leucémie), une tumeur osseuse et un lymphome.

### Immunothérapie cellulaire

Par « traitement anti-tumoral » ou «thérapie anti-tumorale» ou « utilisation thérapeutique anti-tumorale », on entend au sens de l'invention un traitement thérapeutique destiné à réduire le volume, inhiber la croissance, diminuer l'agressivité, modifier les caractéristiques fonctionnelles malignes, et/ou faire disparaitre une tumeur présente dans un organisme.

Pour déterminer et suivre l'efficacité d'un traitement anti-tumoral, un paramètre indicatif est l'évolution de la taille ou du volume de la tumeur au sein de l'organisme, au cours du temps. Chez des animaux de laboratoire, la taille de la tumeur est le plus souvent mesurée après sacrifice des animaux. Chez des patients, la taille de la tumeur pourra être mesurée *in vivo* par des techniques d'imagerie non invasives, bien connues de l'homme du métier.

Comme cela est illustré en figures 1 et 5, la formulation liposomale contenant exclusivement un LPS permet de réduire signification le développement d'une tumeur humaine xenogreffée chez des souris, en comparaison avec le développement tumoral observé chez les souris contrôles, non traitées.

La figure 1 présente les résultats obtenus avec des souris xenogreffées avec des cellules de lymphome RL, et la figure 5 présente les résultats obtenus avec des souris portant des tumeurs de cancer du sein (formées à partir de cellules MDA-MB361).

La présente invention concerne en particulier une formulation liposomale contenant exclusivement un LPS pour son utilisation en tant qu'agent d'immunothérapie.

La présente invention concerne également une formulation liposomale contenant exclusivement un LPS pour son utilisation en tant qu'agent stimulant le système immunitaire inné.

Plus particulièrement, la présente invention concerne une formulation liposomale contenant exclusivement un LPS pour son utilisation en tant qu'agent d'immunothérapie cellulaire anti-tumorale.

L'immunothérapie est une approche thérapeutique consistant à stimuler les fonctions immunitaires internes d'un organisme affecté par un cancer, afin que le système immunitaire de l'organisme soit capable d'inhiber la croissance ou même de faire disparaitre une tumeur en développement en son sein.

Parmi les acteurs immunitaires impliqués dans la reconnaissance et la destruction des cellules cancéreuses, on peut citer les cellules 'Natural Killer' désignées ci-après cellules NK. Il s'agit de lymphocytes capables de reconnaitre un tissu tumoral, de l'infiltrer et d'exercer une cytotoxicité spécifique à l'égard des cellules tumorales.

L'immunothérapie cellulaire anti-tumorale est une approche thérapeutique consistant à stimuler les cellules NK, leur développement et/ou leur activité, afin que celles-ci reconnaissent et lysent les cellules tumorales.

Comme cela est illustré en figures 2 et 3, l'administration à des souris d'une formulation liposomale contenant exclusivement un LPS augmente significativement le nombre des cellules NK présentes dans la rate des animaux.

Ainsi, il apparait qu'une formulation liposomale contenant exclusivement un LPS stimule la multiplication des cellules NK, et agit donc comme un agent d'immunothérapie cellulaire, en favorisant la réponse cellulaire cytotoxique contre les cellules tumorales.

### Formulation liposomale

Au sens de l'invention, une formulation liposomale désigne une composition comprenant des liposomes encapsulant un principe actif, ledit principe actif étant désigné comme étant « encapsulé » ou encore « contenu dans une formulation liposomale ».

Dans la présente demande, les termes « formulation liposomale de LPS », « formulation liposomale contenant exclusivement un LPS », « liposomes-LPS » et « LPS encapsulé dans des liposomes» sont utilisées indifféremment et désignent tous la même formulation telle que définie ci-dessus, à savoir une formulation/composition constituée de liposomes encapsulant un lipopolysacharide bactérien.

Les liposomes, aussi désignés particules liposomales, sont des vésicules dans lesquelles une phase lipidique constituée par une bicouche de molécules amphiphiles, telles que des phospholipides ou du cholestérol, emprisonne une phase interne aqueuse.

On distingue les liposomes unilamellaires, qui comprennent une seule bicouche lipidique, et les liposomes multilamellaires qui comprennent plusieurs bicouches lipidiques concentriques.

Les phospholipides sont des lipides comprenant un groupement 'acide phosphorique'. Ce sont des lipides constitués d'une «tête» polaire (hydrophile) et de deux « queues » aliphatiques (hydrophobes). Cette famille inclut notamment les acides phosphatidiques et les phosphoglycérides. Les propriétés physicochimiques des phospholipides dépendent à la fois de la nature de la molécule polaire de la tête hydrophile, et de la nature des chaînes aliphatiques (acides gras) de leurs queues hydrophobes.

Pour la préparation de la formulation liposomale selon l'invention, différents types de phospholipides peuvent être utilisés.

Par exemple, les formulations liposomales présentées dans la demande de brevet WO 2013/129936 conviennent pour la mise en œuvre de la présente invention.

De manière non exhaustive, les phospholipides suivants pourront être utilisés en combinaison:
- Le 'DOPE' qui désigne le 1,2-Dioleoyl-sn-Glycero-3-Phosphoethanolamine ;
- Le 'DSPE' qui désigne le 1,2-distearoyl-sn-glycero-3-phosphoethanolamine ou le distearoylphosphatidylethanolamine ;
- Le 'PEGXXXX' qui désigne le polyéthylène glycol, avec XXXX indiquant le poids moléculaire de celui-ci ; on utilisera notamment le PEG350 et le PEG5000 ;
- Le 'DSPE-PEGXXXX' qui désigne le 1,2-distearoyl-sn-glycero-3-phosphoethanolamine-N-[methoxy-(polyéthylène glycol)-XXXX].
- Le cholestérol (3β-Hydroxy-5-cholestene, 5-Cholesten-3β-ol).

Selon une mise en œuvre particulière de l'invention, la formulation liposomale est constituée de phospholipides de composition : DOPE: DSPE-PEG 5000: DSPE-PEG-350:Cholestérol (54:8:8:30 mol %).

Selon une autre mise en œuvre particulière de l'invention, la formulation liposomale est constituée de phospholipides de composition :
- 18:1 (delta9-Cis) DSPE (DOPE)
- 18:0 PEG5000 DSPE (sels d'ammonium)
- 18:0 PEG350 DSPE (sels d'ammonium), et
- Cholesterol.

### Lipopolysaccharide bactérien (LPS)

La formulation liposomale selon l'invention contient exclusivement un lipopolysaccharide bactérien, c'est-à-dire ne contient aucun autre composé actif, et en particulier ne contient aucun antigène, à l'exception de l'antigène O constituant du LPS.

Dans le cadre de la présente invention, le LPS est utilisé en tant qu'agent stimulant le système immunitaire inné, et non en tant qu'adjuvant permettant d'augmenter la réponse immunitaire spécifique contre un antigène particulier.

Le LPS, composant de la membrane externe des bactéries à Gram négatif,est composé de trois entités moléculaires liées entre elles par des liaisons covalentes:
- le lipide A;
- le«noyau» constitué d'un oligosaccharide ;
- et l'antigène O.

Le lipide A qui est la partie la plus immunostimulante du LPS est très conservé d'une espèce bactérienne à l'autre, le noyau est très peu variable, et l'antigène O est spécifique de l'espèce bactérienne de provenance du LPS.

De manière non exhaustives, les bactéries à Gram négatif comprennent notamment les familles suivantes :
- Famille des *Enterobacteriaceae* :
   ∘ Genre *Salmonella*
   ∘ Genre *Escherichia,* par exemple Escherichia coli
   ∘ Genre *Yersinia*
- Famille des *Vibrionaceae* :
   ∘ Genre *Vibrio* exemple: *Vibriocholerae* (responsable du choléra)
- Famille des *Pseudomonadaceae* :
   ∘ Genre Pseudomonas
- Famille des Neisseriaceae
   ∘ Genre *Neisseria* exemple: *Neisseria meningitidis* (responsable de la méningite bactérienne)
- Famille des *Rhizobiaceae*
   ∘ Genre *Agrobacterium* exemple: *Agrobacterium tumefaciens*
   ∘ Genre *Rhizobium* exemple: *Rhizobium rhizogenes*
- Famille des *Alcaligenaceae*
   ∘ Genre *Bordetella*

Selon une mise en œuvre particulière de l'invention, le LPS contenu dans la formulation liposomale est issu d'une bactérie de la famille des *Enterobacteriaceae,* à l'exclusion de l'espèce *Salmonella enterica.*

Selon une autre mise en oeuvre particulière de l'invention, le LPS contenu dans la formulation liposomale est issu d'une bactérie de la famille des *Enterobacteriaceae,* notamment du genre *Escherichia,* et en particulier de l'espèce *Escherichia coli.*

L'expression « LPS issu d'une bactérie » indique que ladite molécule de LPS a les caractéristiques physico-chimiques du LPS naturellement présent dans la membrane externe de ladite bactérie. Les caractéristiques sont notamment la nature des acides gras constituant le lipide A.

Le LPS peut être un LPS naturel ou un LPS synthétique.

Plus particulièrement, un LPS naturel peut être purifié à partir d'une membrane bactérienne, par des techniques bien connues de l'Homme du métier, ou encore être obtenu sous une forme purifiée auprès d'une société commerciale telle que SIGMA-ALDRICH.

Un LPS synthétique pourra être obtenu par toute technique de synthèse connue de l'Homme du métier.

Le LPS utilisé peut être encapsulé dans des liposomes sous sa forme entière (lipide A + noyau + antigène O) ou sous une forme incomplète, seule une fraction du LPS étant utilisée.

Selon un aspect particulier de l'invention, le LPS encapsulé est utilisé sous une forme incomplète, c'est-à-dire sous la forme d'une fraction du LPS. Plus particulièrement, le LPS est constitué exclusivement de son antigène O, ou de son lipide A, ou encore d'une combinaison des deux.

Le lipide A du LPS issu de l'espèce bactérienne *Escherichia coli* est un dimère de β-1-6 glucosamine.

Plus particulièrement, le LPS encapsulé peut consister en son lipide A utilisé sous la forme de monophosphoryl lipide A.

Il est entendu que le LPS peut être modifié, notamment afin d'inhiber ou de potentialiser ses propriétés immunostimulantes. Le LPS pourra également être un LPS ayant subi une modification en vue de modifier ses propriétés de solubilité ou de toxicité.

Selon un aspect particulier de l'invention, dans la formulation liposomale pour son utilisation thérapeutique, notamment anti-tumorale, le LPS encapsulé dans les liposomes est un LPS modifié.

### Préparation et caractérisation de la formulation liposomale de LPS

Toute technique connue de l'Homme du métier peut être utilisée pour préparer une formulation liposomale contenant exclusivement un LPS.

Il est entendu que l'expression « exclusivement un LPS» indique que la formulation liposomale comprend uniquement des molécules de LPS, à l'exclusion de tout autre composé actif et/ou antigène.

Le LPS encapsulé est issu d'une certaine espèce bactérienne, par exemple issu *d'Escherichia coli.*

Selon une mise en œuvre particulière de l'invention, ladite formulation comprend exclusivement des molécules de LPS, ces molécules pouvant être de différentes origines bactériennes : ainsi, il peut s'agir d'un, deux, trois voire plus de LPS de différentes origines bactériennes. Par exemple la formulation liposomale peut comprendre un LPS issu *d'E. coli,* un LPS issu d'une bactérie du genre *Salmonella* et un LPS issu d'une bactérie du genre *Pseudomonas,* lesdites molécules de LPS étant sous une forme complète ou incomplète.

Autrement dit, la formulation pharmaceutique consiste en au moins un LPS bactérien encapsulé dans des liposomes.

Comme cela est présenté dans la section des exemples, l'expression « formulation liposomale contenant exclusivement un LPS» indique que le LPS est bien incorporé / encapsulé dans les particules liposomales, et qu'il ne s'agit pas ici d'une simple juxtaposition dans un milieu.

Selon un aspect particulier, le procédé de préparation de la formulation liposomale comprend des étapes successives de congélation / décongélation.

Selon un autre aspect de l'invention, le procédé de préparation de la formulation liposomale comprend une étape de stérilisation de la formulation liposomale.

Selon encore un autre aspect de l'invention, le procédé de préparation de la formulation liposomale comprend une étape de filtration des liposomes, afin d'obtenir des particules liposomales de taille homogène.

Selon un aspect préféré de l'invention, ladite formulation liposomale est constituée de particules liposomales de taille homogène.

Selon une mise en œuvre préférée de l'invention, la formulation liposomale est adaptée pour une administration systémique.

### Composition pharmaceutique

La présente invention concerne également une composition pharmaceutique comprenant, dans un milieu pharmaceutique acceptable, au moins une formulation liposomale contenant exclusivement un lipopolysaccharide.

Autrement dit, ladite composition pharmaceutique comprend, dans un milieu pharmaceutique acceptable, au moins une formulation liposomale consistant en un lipopolysaccharide bactérien encapsulé dans des liposomes.

Au sens de l'invention, un milieu pharmaceutique acceptable désigne un véhicule permettant de conserver et d'administrer la formulation liposomale, et optionnellement des excipients, dont l'administration à un individu ou un animal ne s'accompagne pas d'effets délétères significatifs, et qui sont bien connus de l'homme du métier.

Une composition pharmaceutique selon l'invention peut comprendre tout excipient pharmaceutique nécessaire, tels que des agents tampons ou des agents pour ajuster le pH ou l'isotonicité, ou encore des agents mouillants. Une composition pharmaceutique selon l'invention peut également comprendre un ou plusieurs agents anti-oxydants, et/ou un ou plusieurs agents conservateurs.

Une formulation liposomale, ou une composition pharmaceutique telle que décrite ci-dessus, peuvent être administrés par toute voie convenable, telle que la voie orale, buccale, sublinguale, ophtalmique, rectale, topique, ou par la voie parentérale, notamment par une voie intrapéritonéale, intradermique, sous-cutanée, intraveineuse ou intramusculaire.

Selon une mise en œuvre préférée de l'invention, la formulation liposomale ou la composition pharmaceutique telle que décrite ci-dessus est adaptée pour une administration systémique.

Une composition pharmaceutique selon l'invention peut être formulée pour l'administration par voie orale, sous forme d'un comprimé, d'une capsule ou d'une gélule, à libération prolongée ou contrôlée, d'une pilule, d'une poudre, d'une solution, d'une suspension, d'un sirop ou d'une émulsion.

Selon un autre mode de réalisation, une composition pharmaceutique selon l'invention peut être préparée pour une administration par voie parentérale, sous une forme injectable.

Une composition pharmaceutique selon l'invention peut être stérilisée par toute méthode conventionnelle connue, telle que la filtration. La solution aqueuse résultante peut être conditionnée pour être utilisée en l'état, ou être lyophilisée. Une préparation lyophilisée peut être combinée avec une solution stérile avant utilisation.

Selon une mise en œuvre préférée de l'invention, la composition pharmaceutique comprend une quantité efficace d'une formulation liposomale contenant exclusivement un LPS.

Une quantité efficace d'une telle formulation correspond à une quantité qui induit la réponse désirée, à savoir un effet thérapeutique, et plus spécifiquement un effet antiprolifératif à l'égard des cellules tumorales. La quantité efficace peut dépendre d'un paramètre ou d'une pluralité de paramètres, tel que la voie d'administration, l'administration en dose unique ou multiple, les caractéristiques du patient, ce qui englobe l'âge, la condition physique, la taille, le poids et la présence d'affections en plus de celle traitée. Ces paramètres et leurs influences sont bien connus de l'homme de l'art et peuvent être déterminés par toute méthode connue.

La présente invention concerne également ladite composition pharmaceutique, pour son utilisation en tant que médicament, et plus particulièrement pour son utilisation thérapeutique anti-tumorale.

### Administration

La formulation liposomale contenant exclusivement un LPS, ou une composition pharmaceutique la comprenant, pour son utilisation thérapeutique anti-tumorale, pourra être administrée à un patient atteint d'un cancer selon toutes les techniques connues de l'Homme du métier.

En particulier, ladite formulation liposomale ou composition pharmaceutique la comprenant pourront être administrées en une seule prise, ou en plusieurs fois sur une période continue.

Selon un aspect particulier de l'invention, la formulation liposomale pour son utilisation telle que décrite ci-dessus, ou une composition pharmaceutique la comprenant, est administrée une fois par semaine à un patient atteint d'un cancer.

Selon une mise en œuvre préférée de l'invention, ladite formulation liposomale pour son utilisation telle que décrite ci-dessus est administrée par une voie systémique, c'est à dire que la formulation empruntera la voie sanguine du patient pour atteindre ses cellules cibles. Plus précisément, ladite formulation liposomale pourra être administrée par la voie digestive ou par la voie parentérale.

La présente invention concerne également une méthode de traitement thérapeutique d'une tumeur, comprenant l'administration à un patient présentant ladite tumeur, d'une quantité efficace d'une formulation liposomale contenant exclusivement un LPS.

### Produit de combinaison pharmaceutique

Selon un troisième aspect, l'invention concerne un produit de combinaison pharmaceutique comprenant :
- une formulation liposomale contenant exclusivement un LPS ; et
- au moins un composé cytotoxique.

Au sens de l'invention, un produit de combinaison pharmaceutique désigne un ensemble d'agents thérapeutiques utilisés conjointement pour le traitement d'une même pathologie, leur administration pouvant être simultanée, séparée ou séquentielle. Ainsi, les agents thérapeutiques peuvent être soit mélangés dans une même composition thérapeutique, soit présents dans un même kit mais administrés de façon complètement séparée, ou séquentielle.

La formulation liposomale contenant exclusivement un LPS sera telle que définie précédemment, et pourra notamment être :
- Une formulation contenant un LPS issu d'une bactérie de la famille des *Enterobacteriaceae,* notamment du genre *Escherichia,* et en particulier de l'espèce *Escherichia coli* ; et/ou
- Une formulation contenant un LPS constitué exclusivement de son lipide A.

Comme précédemment évoqué, une formulation liposomale de LPS peut être utilisée en thérapie soit en tant que telle, soit être combinée à un autre agent thérapeutique. Cet autre agent thérapeutique est en particulier un composé cytotoxique, c'est-à-dire un composé induisant la mort cellulaire des cellules sur lesquelles il agit.

Avantageusement, les composés cytotoxiques sont adaptés pour cibler spécifiquement certaines cellules, notamment pour cibler les cellules cancéreuses.

Selon une mise en œuvre de l'invention, ledit composé cytotoxique est un agent d'immunothérapie, tels que des peptides ou des petites molécules non peptidiques présentant une activité immuno-modulatrice et cytotoxique; plus préférentiellement l'agent d'immunothérapie est un anticorps thérapeutique.

Un anticorps thérapeutique est un anticorps capable de reconnaitre spécifiquement les cellules destinées à être détruites, dans le cas d'un cancer il s'agit naturellement des cellules tumorales. Les cellules tumorales expriment des antigènes à leur surface membranaire, qui peuvent être reconnus par des anticorps dirigés contre ceux-ci. Les anticorps thérapeutiques peuvent être couplés à une substance toxique, capables d'induire la lyse et donc la mort de la cellule tumorale reconnue par l'anticorps. Les anticorps thérapeutiques peuvent également agir en bloquant certains récepteurs à la surface membranaire des cellules tumorales.

Ces anticorps thérapeutiques sont de plus en plus utilisés dans le cadre de thérapies anti-tumorales. On peut citer par exemple le rituximab, un anticorps monoclonal se liant spécifiquement à l'antigène transmembranaire CD20, une protéine située sur les lymphocytes B et s'exprimant dans plus de 95 % des cellules B des lymphomes non hodgkiniens. Cet anticorps thérapeutique est indiqué pour le traitement de patients atteints de lymphomes folliculaires de stade III-IV. On peut citer également le trastuzumab, spécifique du récepteur HER-2 surexprimé par certaines cellules tumorales de cancer de sein.

Selon une mise en oeuvre particulière de l'invention, l'anticorps thérapeutique contenu dans le produit de combinaison est un anticorps monoclonal.

Selon une autre mise en oeuvreparticulière de l'invention, l'anticorps thérapeutique contenu dans le produit de combinaison est un anticorps polyclonal.

Selon une mise en oeuvre de l'invention, la cible de l'anticorps thérapeutique est choisie parmi le goupe consistant en : CD20, CD52, CD3, CD4, CD5, CD8, CD19, CD22, CD38, CD138, HER2, ErbB2, CD1, CD30, CD33, CD52, CD25, le facteur de croissance vasculaire endothélial (VEGF), le récepteur du facteur de croissance endothéliale (EGFR), le récepteur du facteur de croissance Insuline-like 1 (IGF1) et CTLA-4.

Selon encore une autre mise en œuvre de l'invention, l'anticorps thérapeutique est choisi parmi le groupe d'anticorps consistant en : abciximab, adalimumab, alemtuzumab, atlizumab, basiliximab, belimumab, bevacizumab, brentuximabvedotin, canakinumab, cetuximab, certolizumabpegol, cixutumumab, daclizumab, denosumab, eculizumab, efalizumab, gemtuzumab, golimumab, ibritumomab tiuxetan, infliximab, ipilimumab (MDX-101), muromonab-CD3, natalizumab, necitumunab, obinutuzumab (GA-101), ocaratuzumab (AME-133v), ocrelizumab, ofatumumab, omalizumab, palivizumab, panitumumab, pertuzumab, PR0131921, ranibizumab, rituximab, SBI-087, tocilizumab, TRU-015, tositumomab, trastuzumab, veltuzumab, et toute combinaison de ces anticorps entre eux.

Il est entendu qu'au sens de l'invention, le produit de combinaison contient au moins un anticorps thérapeutique, et qu'il peut donc comprendre une combinaison de plusieurs anticorps thérapeutiques.

Selon une autre mise en œuvre de l'invention, l'anticorps thérapeutique est choisi parmi le groupe consistant en des anticorps utilisés dans des applications thérapeutiques anti-tumorales, et notamment consistant en les anticorps suivants: alemtuzumab, belimumab, bevacizumab, brentuximab vedotin, canakinumab, cetuximab, cixutumumab, daclizumab, denosumab, gemtuzumab, golimumab, ibritumomab tiuxetan, ipilimumab (MDX-101), muromonab-CD3, natalizumab, necitumumab, obinutuzumab (GA-101), ocaratuzumab (AME-133v), ocrelizumab, ofatumumab, panitumumab, pertuzumab, PR0131921, ranibizumab, rituximab, SBI-087, tocilizumab, TRU-015, tositumomab, trastuzumab, veltuzumab, et toute combinaison de ces anticorps entre eux.

Selon une mise en oeuvre préférée de l'invention, l'anticorps thérapeutique est un anticorps monoclonal anti-CD20, et de manière préférée est le rituximab.

Les résultats expérimentaux présentés en figure 1 montrent que :
- chez des souris présentant une tumeur, non traitées, le volume de la tumeur augmente au cours du temps ;
- si les souris sont traitées par administration de rituximab, le volume de la tumeur augmente toujours au fil du temps, mais ledit volume est deux fois moins important, 46 jours après la greffe de tumeur, que le volume de la tumeur chez des souris contrôles ;
- si les souris sont traitées avec un produit de combinaison comprenant du rituximab et une formulation liposomale de LPS, le volume de la tumeur est identique à 23 jours et à 46 jours après la greffe : le développement de la tumeur est donc complètement inhibé.

La figure 4 présente la différence observée entre des souris traitées avec ce produit de combinaison, et des souris traitées avec un produit de combinaison composé de rituximab et de LPS non encapsulé dans des liposomes : la formulation liposomale de LPS est plus efficace pour inhiber le développement de la tumeur.

Selon une autre mise en œuvre de l'invention, le composé cytotoxique est choisi parmi les composés suivants :
- des agents cytotoxiques conventionnels (agents de chimiothérapie) tels que :
   - les agents alkylants et assimilés tels que le cyclophosphamide, le busulfan, le melphalan, l'ifosfamide, la bendamustine, le temozolomide, la bléomycine, la mechloretamine, la carmustine, la fotemustine, le BCNU ;
   - Les dérivés du platine tels que le cisplatine, l'oxaliplatine, le carboplatine,
   - les analogues de nucléosides, nucléobases et autres antimétabolites tels que l'aracytine, la fludarabine, la cladribine, la clofarabine, la gemcitabine, le 5 fluorouracile, la capécitabine, le méthotrexate, le pémétrexed, la 6 mercaptopurine, la decitabine, l'azacytidine, la troxacitabine,
   - les agents antitubuline tels que la vincristine, la vinblastine, la vindesine, la vinorelbine, la vinflunine, le paclitaxel, le docetaxel, le cabazitabel, les épothilones, l'éribuline, le paclitaxel micronisé,
   - les agents anti topoisomérases 2 tels que l'étoposide, le téniposide, l'irinotecan, la camptothécine, la doxorubicine, la daunorubicine, l'idarubicine, la mitoxantrone, la dactinomycine;
- des agents de thérapeutiques ciblées de type inhibiteurs de kinases tels que l'imabinib, le dasatinib, le bosutinib, le nilotinib, le ponatinib, l'ibrubinib, l'idelalisib, l'afatinib, l'erlotinib, le gefitinib, le lapatinib, le crizotinib, le ruxolitinib, le tofacitinib, l'axitinib, le cabozantinib, le nindetanib, le pazopanib, le vandetanib, le regorafenib, le sorafenib, le sunitinib, le dabrafenib, le trametinib, le vemurafenib ;
- Les immunomodulateurs tels que la thalidomide, le lenalidomide, le pomalidomide ;
- Les facilitateurs d'apoptose tels que le venetoclax ou le navitoclax ;
- D'autres agents à propriété antitumorale tels que le bortezomib, le carfilzomib, l'ixazomib, le vorinostat, le panobinostat, la romidepsine, l'acide tout trans rétinoïque, le dérivé d'arsenic As2O3, le temsirolimus, l'olaparib, le rucaparib, la pentostatine, l'asparaginase, l'hydroxyurée et la chloraminophène.

Selon une mise en œuvre préférée de l'invention, le composé cytotoxique est un agent de chimiothérapie.

Il est entendu que les composés cités ci-dessus pourront être utilisés sous leur forme habituelle, en particulier sous forme de sels. Par exemple, l'étoposide pourra être utilisé sous la forme de phosphate d'épotoside.

L'exemple 4 de la présente demande démontre que du LPS encapsulé dans des liposomes, utilisé en combinaison avec de l'étoposide (5 µM), permet d'induire la mort cellulaire de cellules cancéreuses *in vitro,* selon un mode synergique.

En effet, l'étoposide, agent de chimiothérapie classiquement utilisé, induit l'apoptose des cellules cancéreuses, comme attendu ; par ailleurs, la formulation liposomale de LPS stimule les macrophages, induisant ainsi une lyse des cellules tumorales ; et l'effet combiné des deux composants permet d'obtenir un taux de mort cellulaire bien supérieur à la somme des deux effets de chaque composant utilisé de façon séparé.

La présente invention concerne aussi un produit de combinaison pharmaceutique tel que présenté ci-dessus, pour son utilisation en thérapie anti-tumorale

En particulier, ledit produit de combinaison pharmaceutique sera utilisé pour traiter une tumeur liquide ou une tumeur solide.

Ladite tumeur pourra notamment être choisie parmi le groupe consistant en : une tumeur de sein, de poumon, de peau (mélanome), de sang (leucémie), une tumeur osseuse et un lymphome.

Plus précisément, la présente invention concerne ledit produit de combinaison pharmaceutique pour son utilisation simultanée, séparée ou séquentielle en thérapie anti-tumorale. Autrement dit, l'utilisation de chaque composant dudit produit de combinaison pourra être simultanée, séparée ou séquentielle.

Plus particulièrement, elle concerne un produit de combinaison pharmaceutique tel que présenté ci-dessus, pour son utilisation simultanée, séparée ou séquentielle en immunothérapie anti-tumorale.

La présente invention concerne également une méthode de traitement thérapeutique d'une tumeur, comprenant l'administration à un patient présentant ladite tumeur, d'au moins un composé cytotoxique et d'une formulation liposomale contenant exclusivement un LPS, ladite administration étant réalisée de manière simultanée, séparée ou séquentielle.

La présente invention est aussi relative à un kit comprenant :
- une formulation liposomale contenant exclusivement un LPS ; et
- au moins un composé cytotoxique tel que défini ci-dessus.

Ledit composé cytotoxique pourra notamment être un agent de chimiothérapie ou un anticorps thérapeutique.

### EXEMPLES

### Exemple 1. Préparation, analyse et caractérisation des formulations liposomales dans lesquelles les molécules de LPS (LPS extrait et purifié à partir de d'Escherichia coli 055:B5) sont encapsulées : liposomes-LPS

Les formulations de liposomes-LPS ont été synthétisées selon la procédure suivante :
- Composition lipidique de la formulation liposomale : DOPE: DSPE-PEG 5000: DSPE-PEG-350:Chol (54:8:8:30 mol %)
- Dissolution des lipides dans un mélange de chloroforme/méthanol (9:1)
- Préparation du film lipidique après évaporation des solvants organiques. L'évaporation est effectuée à l'aide d'un évaporateur rotatif
- L'hydratation du film lipidique est effectuée en ajoutant du PBS (buffered saline solution) contenant 100 µg de LPS/ml dans le ballon placé sur un évaporateur rotatif, sans vide.
- Pour améliorer le taux d'encapsulation une série de congélation (dans l'azote liquide) - décongélation (dans l'eau tiède) est appliquée à la suspension de liposomes-LPS.

L'extrusion de la solution de liposomes-LPS permet d'obtenir des liposomes de taille homogène. Pour cela les solutions de liposomes-LPS sont filtrées sur des membranes de polycarbonate de 800, 400 et 200 nm. Après 5 passages sur chaque filtre la taille des liposomes est homogène et la solution de liposomes-LPS est translucide.

L'analyse de la taille (diamètre moyen), de la polydispersité (PDI) et de la charge des préparations liposomales a été effectuée par diffusion dynamique de lumière à l'aide d'un ZetasizerNano-S de Malvern instruments (Worcestershire, UK). L'analyse sur 3 mois de deux préparations liposomales a permis de démontrer la stabilité de ces préparations (tableau 1).

**Tableau 1 :**

| | Mesures faites à | Taille (nm) | Polydispersité (PDI) | Osmolarité (mOsm/kg) | Potentiel Zeta (mv) |
|---|---|---|---|---|---|
| Préparation liposomale contenant du LPS | J0 | 133 | 0,09 | 309 | -3,63 |
| | J0 + 30 j | 132 | 0,09 | 308 | -3,67 |
| | J0 + 60 j | 133 | 0,111 | 311 | ND |
| | J0 + 90 j | 134 | 0,098 | 310 | -6,13 |

L'analyse *in vitro* de l'interaction des formulations liposomales ainsi produites (faisant appel à un composant fluorescent) avec les cellules leucocytaires du sang humain nous a permis de démontrer une forte interaction des liposomes avec les granulocytes et les monocytes. L'interaction avec la fraction lymphocytaire n'a pas été observée (résultats non montrés).

Par ailleurs, l'incubation des leucocytes du sang humain avec les liposomes-LPS et l'analyse par cytométrie en flux nous a permis de mettre en évidence l'activation de l'activité phagocytaire.

L'analyse en microscopie confocale des formulations liposomes-LPS marquées à la rhodamine B et dans lesquelles les molécules LPS sont couplées au fluorochrome FITC nous a permis de mettre en évidence l'incorporation du LPS dans les particules liposomales.

Enfin, l'homogénéité des particules liposomales a été validée par microscopie électronique après coloration négative.

### Exemple 2. Evaluation préclinique de l'effet adjuvant des formulations liposomales de LPS sur l'activité du rituximab dans un modèle de souris porteuses de xénogreffes de cellules RL

Au cours de cette expérience, six groupes de souris Scid CB17ont été utilisés. Chaque groupe est composé de six souris.
Groupe 1 = contrôle (aucun traitement)
Groupe 2 = Liposomes vides
Groupe 3 = LPS 0,5 mg/ml
Groupe 4 = Rituximab 30mg/kg
Groupe 5 = Liposomes-LPS 0,5 mg/kg
Groupe 6 = Liposomes-LPS 0,5 mg/kg + Rituximab 30mg/kg

Le suivi de croissance tumorale après l'injection des différents traitements à raison d'une injection par semaine a permis de démontrer l'effet potentialisateur de l'activité antitumorale du rituximab par la préparation de liposomes-LPS. Cet effet potentialisateur s'est traduit par une réduction significative de la croissance tumorale après la combinaison du Rituximab et des liposomes-LPS en comparaison avec le groupe de souris traité par le rituximab seul (Figure 1).

L'analyse de l'effet des différents traitements sur la taille de la rate après sacrifice des animaux a permis la mise en évidence d'une augmentation significative de la taille et du poids de la rate dans le cas des animaux traités par les liposomes-LPS et la combinaison du Rituximab et Liposomes-LPS en comparaison avec les animaux des autres groupes (figure 2).

Les cellules NK sont des cellules de l'immunité innée qui sont connues pour jouer un rôle important dans les réponses antitumorales.

L'analyse par cytométrie en flux de l'effet des différents traitements sur les sous populations leucocytaires au niveau de la rate a permis la mise en évidence d'une augmentation significative du nombre de cellules Natural Killer (NK) dans la rate des animaux traités par les liposomes-LPS et la combinaison du Rituximab et Liposomes-LPS en comparaison avec les animaux des autres groupes (la figure 3 présente le pourcentage des cellules NK par rapport à l'ensemble des cellules leucocytaires dans l'échantillon).

La figure 4 présente, dans le même modèle animal de souris SCID greffées avec des cellules de lymphome RL, les effets comparés obtenus avec l'administration, une fois par semaine, de :
- rituximab (30 mg/kg)
- rituximab et LPS « conventionnel » *d'E. coli* (0,5 mg/kg)
- rituximab et formulation liposomale de LPS, aux mêmes doses que précédemment.

Cette dernière combinaison de rituximab et LPS encapsulé permet d'obtenir la meilleure inhibition du développement de la tumeur après sa greffe.

### Exemple 3. Action antitumorale de la formulation liposomale de LPS dans des modèles de xénogreffes de tumeurs solides

L'action anti-tumorale de la formulation liposomale de LPS a également été montrée dans un autre modèle animal de tumeurs, des souris SCID xénogreffées avec des cellules de tumeurs de cancer du sein : les cellules MDA-MB-231.

Les résultats présentés en figure 5 montrent l'évolution du volume de la tumeur au cours du temps, en fonction des traitements administrés aux souris :
- Groupe 1 : souris contrôles non traitées (3 souris) ;
- Groupe 2 : souris traitées par administration intraveineuse de la formulation liposomale de LPS.

Le suivi de croissance tumorale après l'injection des différents traitements à raison d'une injection par semaine a permis de démontrer l'activité anti-tumorale de la préparation de liposomes-LPS. Cette activité s'est traduite par une réduction significative de la croissance tumorale après injection des liposomes-LPS en comparaison avec le groupe de souris non traitées.

### Exemple 4. Effets in vitro d'un produit de combinaison selon l'invention sur le taux de mort cellulaire de cellules SaOS

Les cellules SaOS sont des cellules d'ostéosarcome de rat. Il s'agit d'une tumeur maligne osseuse. Ces cellules peuvent être cultivées selon un modèle sphéroïde en 3D adapté aux cellules tumorales.

Les THP-1 sont des cellules monocytaires humaines dérivées d'une leucémie monocytaire aiguë.

Pour effectuer le suivi de la mort cellulaire de ces cellules SaOS, le produit fluorescent IncuCyte^{®} Caspase-3/7 a été utilisé pour évaluer le nombre de cellules en apoptose.

Les cellules ont été traitées selon le protocole présenté dans le tableau 2 suivant :

| **Jours** | **Cellules THP-1 effectrices** | **Cellules Saos cibles** |
|---|---|---|
| **J0** | Activation des cellules THP-1 en macrophages : traitement avec 150 nM de PMA (acétate de phorbol myristate) pendant 24 heures | Les cellules Saos sont placées dans des plaques 96 puits (2500 cellules / puits) pour initier la croissance des sphéroïdes |

| **Jours** | **Cellules THP-1 effectrices** | **Cellules Saos cibles** |
|---|---|---|
| **J1** | Changement de milieu de culture | |
| **J2** | Activation des THP-1 avec une incubation pendant 16 heures en présence de Lipo-LPS 100 ng/ml. | |
| **J3** | | Mise en présence des cellules THP-1 activées et des cellules cibles : |
| | | - Ratio cibles/effectrices = 1/3 |
| | | - + étoposide 5 µM |
| | | - + Composé fluorescent Green casp 3/7 à 2,5 mM final |
| | | Incubation 30 minutes à température ambiante puis observation au microscope après révélation par Incucyte^{®}, toutes les trois heures |

### Tableau 2. Protocole de traitement des cellules effectrices THP-1 et des cellules SaOS cibles

Les résultats sont présentés en Figure 6, en unités arbitraires de fluorescence.

Les cellules contrôles présentent un faible taux d'apoptose, résiduel.

Les cellules traitées avec de l'épotoside présente un taux d'apoptose significativement augmenté par rapport au contrôle (p<0,05). Les cellules traitées avec une formulation liposomale de LPS présentent un taux similaire.

L'association des deux composés : épotoside et formulation liposomale, permet d'obtenir un taux d'apoptose significativement augmenté par rapport à celui obtenu pour chacun des composés utilisé isolément, démontrant ainsi l'effet synergique de ces deux composés sur les cellules cancéreuses cibles.

### REFERENCES

### BREVET

WO 2013/129936

### REFERENCES BIBLIOGRAPHIQUES

Bakouche O, Koff WC, Brown DC, Lachman LB. Interleukin 1 release by human monocytes treated with liposome-encapsulated lipopolysaccharide. J. Immunol. 1987 Aug 15; 139(4):1120-6.
Dijkstra J, Mellors JW, Ryan JL. Altered in vivo activity of liposome-incorporated lipopolysaccharide and lipid A. Infect Immun. 1989 Nov; 57(11):3357-63.
Neidhart J, Allen KO, Barlow DL, Carpenter M, Shaw DR, Triozzi PL, Conry RM. Immunization of colorectal cancer patients with recombinant baculovirus-derived KSA (Ep-CAM) formulated with monophosphoryl lipid A in liposomal emulsion, with and without granulocyte-macrophage colony-stimulating factor. Vaccine. 2004 Jan 26; 22(5-6):773-80.

## Revendications

1. Produit de combinaison pharmaceutique comprenant :
- une formulation liposomale contenant exclusivement un lipopolysaccharide bactérien (LPS); et
- au moins un composé cytotoxique.

2. Produit de combinaison pharmaceutique selon la revendication 1, **caractérisé en ce que** le LPS contenu dans la formulation liposomale est issu d'une bactérie de la famille des *Enterobacteriaceae,* notamment du genre *Escherichia,* et en particulier de l'espèce *Escherichia coli.*

3. Produit de combinaison pharmaceutique selon l'une des revendications 1 ou 2, **caractérisé en ce que** le LPS est constitué exclusivement de son lipide A.

4. Produit de combinaison pharmaceutique selon l'une des revendications 1 à 3, **caractérisé en ce que** le composé cytotoxique est un anticorps thérapeutique.

5. Produit de combinaison pharmaceutique selon la revendication 4, dans lequel la cible de l'anticorps thérapeutique est choisie parmi le groupe consistant en : CD20, CD52, CD3, CD4, CD5, CD8, CD19, CD22, CD38, CD138, HER2, ErbB2, CD1, CD30, CD33, CD52, CD25, le facteur de croissance vasculaire endothélial (VEGF), le récepteur du facteur de croissance endothéliale (EGFR), le récepteur du facteur de croissance Insuline-like 1 (IGF1) et CTLA-4.

6. Produit de combinaison pharmaceutique selon la revendication 4 ou 5, dans lequel l'anticorps thérapeutique est choisi parmi le groupe d'anticorps consistant en : abciximab, adalimumab, alemtuzumab, atlizumab, basiliximab, belimumab, bevacizumab, brentuximab vedotin, canakinumab, cetuximab, certolizumab pegol, cixutumumab, daclizumab, denosumab, eculizumab, efalizumab, gemtuzumab, golimumab, ibritumomab tiuxetan, infliximab, ipilimumab (MDX-101), muromonab-CD3, natalizumab, necitumumab, obinutuzumab (GA-101), ocaratuzumab (AME-133v), ocrelizumab, ofatumumab, omalizumab, palivizumab, panitumumab, pertuzumab, PR0131921, ranibizumab, rituximab, SBI-087, tocilizumab, TRU-015, tositumomab, trastuzumab, veltuzumab, et toute combinaison de ces anticorps entre eux.

7. Produit de combinaison pharmaceutique selon l'une des revendications 1 à 3, **caractérisé en ce que** le composé cytotoxique est choisi parmi :
- Des agents de chimiothérapie tels que : le cyclophosphamide, le busulfan, le melphalan, l'ifosfamide, la bendamustine, le temozolomide, la bléomycine, la mechloretamine, la carmustine, la fotemustine, le BCNU, le cisplatine, l'oxaliplatine, le carboplatine, l'aracytine, la fludarabine, la cladribine, la clofarabine, la gemcitabine, le 5 fluorouracile, la capécitabine, le méthotrexate, le pémétrexed, la 6 mercaptopurine, la decitabine, l'azacytidine, la troxacitabine, la vincristine, la vinblastine, la vindesine, la vinorelbine, la vinflunine, le paclitaxel, le docetaxel, le cabazitabel, les épothilones, l'éribuline, le paclitaxel micronisé, l'étoposide, le téniposide, l'irinotecan, la camptothécine, la doxorubicine, la daunorubicine, l'idarubicine, la mitoxantrone, la dactinomycine ;
- Des agents de thérapeutiques ciblées de type inhibiteurs de kinases tels que : l'imabinib, le dasatinib, le bosutinib, le nilotinib, le ponatinib, l'ibrubinib, l'idelalisib, l'afatinib, l'erlotinib, le gefitinib, le lapatinib, le crizotinib, le ruxolitinib, le tofacitinib, l'axitinib, le cabozantinib, le nindetanib, le pazopanib, le vandetanib, le regorafenib, le sorafenib, le sunitinib, le dabrafenib, le trametinib, le vemurafenib ;
- Des immunomodulateurs tels que la thalidomide, le lenalidomide, le pomalidomide ;
- Des facilitateurs d'apoptose tels que le venetoclax ou le navitoclax ;
- D'autres agents à propriété antitumorale tels que le bortezomib, le carfilzomib, l'ixazomib, le vorinostat, le panobinostat, la romidepsine, l'acide tout trans rétinoïque, le dérivé d'arsenic As2O3, le temsirolimus, l'olaparib, le rucaparib, la pentostatine, l'asparaginase, l'hydroxyurée, et la chloraminophène.

8. Produit de combinaison pharmaceutique selon la revendication 7, **caractérisé en ce que** le composé cytotoxique est un agent de chimiothérapie.

9. Produit de combinaison pharmaceutique selon l'une des revendications 1 à 8, pour son utilisation thérapeutique anti-tumorale.

10. Produit de combinaison pharmaceutique pour son utilisation selon la revendication 9, **caractérisée en ce que** la tumeur est une tumeur liquide ou une tumeur solide.

11. Produit de combinaison pharmaceutique pour son utilisation selon l'une des revendications 9 ou 10, **caractérisée en ce que** la tumeur est choisie parmi le groupe consistant en : une tumeur de sein, de poumon, de peau (mélanome), de sang (leucémie), une tumeur osseuse et un lymphome.

12. Produit de combinaison pharmaceutique pour son utilisation selon l'une des revendications 9 à 11, **caractérisée en ce que** l'utilisation de chaque composant est simultanée, séparée ou séquentielle.

13. Produit de combinaison pharmaceutique pour son utilisation selon l'une des revendications 9 à 12, **caractérisée en ce que** la formulation liposomale est adaptée pour une administration systémique.

## Patentansprüche

1. Pharmazeutisches Kombinationsprodukt, umfassend:
- eine liposomale Formulierung, die ausschließlich ein bakterielles Lipopolysaccharid (LPS) enthält; und
- mindestens eine zytotoxische Verbindung.

2. Pharmazeutisches Kombinationsprodukt nach Anspruch 1, **dadurch gekennzeichnet, dass** das in der liposomalen Formulierung enthaltene LPS von einer Bakterie der Familie der *Enterobacteriaceae,* besonders der Gattung *Escherichia* und insbesondere der Spezies *Escherichia coli* stammt.

3. Pharmazeutisches Kombinationsprodukt nach einem der Ansprüche 1 oder 2, **dadurch gekennzeichnet, dass** das LPS ausschließlich aus einem Lipid A besteht.

4. Pharmazeutisches Kombinationsprodukt nach einem der Ansprüche 1 bis 3, **dadurch gekennzeichnet, dass** die zytotoxische Verbindung ein therapeutischer Antikörper ist.

5. Pharmazeutisches Kombinationsprodukt nach Anspruch 4, wobei das Ziel des therapeutischen Antikörpers ausgewählt ist aus der Gruppe bestehend aus: CD20, CD52, CD3, CD4, CD5, CD8, CD19, CD22, CD38, CD138, HER2, ErbB2, CD1, CD30, CD33, CD52, CD25, dem vaskulären endothelialen Wachstumsfaktor (VEGF), dem endothelialen Wachstumsfaktorrezeptor (EGFR), dem insulinähnlichen Wachstumsfaktorrezeptor 1 (IGF1) und CTLA-4.

6. Pharmazeutisches Kombinationsprodukt nach Anspruch 4 oder 5, wobei der therapeutische Antikörper ausgewählt ist aus der Antikörpergruppe bestehend aus: Abciximab, Adalimumab, Alemtuzumab, Atlizumab, Basiliximab, Belimumab, Bevacizumab, Brentuximab-Vedotin, Canakinumab, Cetuximab, Certolizumab-Pegol, Cixutumumab, Daclizumab, Denosumab, Eculizumab, Efalizumab, Gemtuzumab, Golimumab, Ibritumomab-Tiuxetan, Infliximab, Ipilimumab (MDX-101), Muromonab-CD3, Natalizumab, Necitumumab, Obinutuzumab (GA-101), Ocaratuzumab (AME-133v), Ocrelizumab, Ofatumumab, Omalizumab, Palivizumab, Panitumumab, Pertuzumab, PR0131921, Ranibizumab, Rituximab, SBI-087, Tocilizumab, TRU-015, Tositumomab, Trastuzumab, Veltuzumab und jeglicher Kombination dieser Antikörper miteinander.

7. Pharmazeutisches Kombinationsprodukt nach einem der Ansprüche 1 bis 3, **dadurch gekennzeichnet, dass** die zytotoxische Verbindung ausgewählt ist aus:
- Chemotherapeutika wie: Cyclophosphamid, Busulfan, Melphalan, Ifosfamid, Bendamustin, Temozolomid, Bleomycin, Mechlorethamin, Carmustin, Fotemustin, BCNU, Cisplatin, Oxaliplatin, Carboplatin, Aracytin, Fludarabin, Cladribin, Clofarabin, Gemcitabin, 5-Fluoruracil, Capecitabin, Methotrexat, Pemetrexed, 6-Mercaptopurin, Decitabin, Azacytidin, Troxacitabin, Vincristin, Vinblastin, Vindesin, Vinorelbin, Vinflunin, Paclitaxel, Docetaxel, Cabazitabel, Epothilone, Eribulin, mikronisiertes Paclitaxel, Etoposid, Teniposid, Irinotecan, Camptothecin, Doxorubicin, Daunorubicin, Idarubicin, Mitoxantron, Dactinomycin;
- zielgerichtete Therapeutika vom Kinasehemmer-Typ wie: Imabinib, Dasatinib, Bosutinib, Nilotinib, Ponatinib, Ibrubinib, Idelalisib, Afatinib, Erlotinib, Gefitinib, Lapatinib, Crizotinib, Ruxolitinib, Tofacitinib, Axitinib, Cabozantinib, Nindetanib, Pazopanib, Vandetanib, Regorafenib, Sorafenib, Sunitinib, Dabrafenib, Trametinib, Vemurafenib;
- Immunmodulatoren wie Thalidomid, Lenalidomid, Pomalidomid;
- Apoptosevermittlern wie Venetoclax oder Navitoclax;
- andere Mittel mit antitumoralen Eigenschaften wie Bortezomib, Carfilzomib, Ixazomib, Vorinostat, Panobinostat, Romidepsin, vollständig trans-Retinsäure, Arsenderivat As₂O₃, Temsirolimus, Olaparib, Rucaparib, Pentostatin, Asparaginase, Hydroxyharnstoff und Chloraminophen.

8. Pharmazeutisches Kombinationsprodukt nach Anspruch 7, **dadurch gekennzeichnet, dass** die zytotoxische Verbindung ein Chemotherapeutikum ist.

9. Pharmazeutisches Kombinationsprodukt nach einem der Ansprüche 1 bis 8 zur therapeutischen antitumoralen Verwendung.

10. Pharmazeutisches Kombinationsprodukt zur Verwendung nach Anspruch 9, **dadurch gekennzeichnet, dass** der Tumor ein liquider Tumor oder ein solider Tumor ist.

11. Pharmazeutisches Kombinationsprodukt zur Verwendung nach einem der Ansprüche 9 oder 10, **dadurch gekennzeichnet, dass** der Tumor ausgewählt ist aus der Gruppe bestehend aus: einem Brust-, Lungen-, Haut- (Melanom), Bluttumor (Leukämie), einem Knochentumor und einem Lymphom.

12. Pharmazeutisches Kombinationsprodukt zur Verwendung nach einem der Ansprüche 9 bis 11, **dadurch gekennzeichnet, dass** die Verwendung jedes Bestandteils gleichzeitig, separat oder sequentiell ist.

13. Pharmazeutisches Kombinationsprodukt zur Verwendung nach einem der Ansprüche 9 bis 12, **dadurch gekennzeichnet, dass** die liposomale Formulierung für eine systemische Verabreichung angepasst wird.

## Claims

1. A pharmaceutical combination product comprising:
- a liposomal formulation exclusively containing a bacterial lipopolysaccharide (LPS); and
- at least one cytotoxic compound.

2. The pharmaceutical combination product as claimed in claim 1, **characterized in that** the LPS contained in the liposomal formulation is from a bacterium of the family *Enterobacteriaceae,* in particular of the genus *Escherichia,* and in particular of the species *Escherichia coli.*

3. The pharmaceutical combination product as claimed in one of claims 1 or 2, **characterized in that** the LPS exclusively consists of its lipid A.

4. The pharmaceutical combination product as claimed in one of claims 1 to 3, **characterized in that** the cytotoxic compound is a therapeutic antibody.

5. The pharmaceutical combination product as claimed in claim 4, wherein the therapeutic antibody target is selected from the group consisting of: CD20, CD52, CD3, CD4, CD5, CD8, CD19, CD22, CD38, CD138, HER2, ErbB2, CD1, CD30, CD33, CD52, CD25, vascular endothelial growth factor (VEGF), endothelial growth factor receptor (EGFR), insulin-like growth factor receptor 1 (IGF1) and CTLA-4.

6. The pharmaceutical combination product as claimed in claim 4 or 5, wherein the therapeutic antibody is selected from the group of antibodies consisting of: abciximab, adalimumab, alemtuzumab, atlizumab, basiliximab, belimumab, bevacizumab, brentuximab vedotin, canakinumab, cetuximab, certolizumab pegol, cixutumumab, daclizumab, denosumab, eculizumab, efalizumab, gemtuzumab, golimumab, ibritumomab tiuxetan, infliximab, ipilimumab (MDX-101), muromonab-CD3, natalizumab, necitumumab, obinutuzumab (GA-101), ocaratuzumab (AME-133v), ocrelizumab, ofatumumab, omalizumab, palivizumab, panitumumab, pertuzumab, PR0131921, ranibizumab, rituximab, SBI-087, tocilizumab, TRU-015, tositumomab, trastuzumab, veltuzumab, and any combination of these antibodies to each other.

7. The pharmaceutical combination product as claimed in one of claims 1 to 3, **characterized in that** the cytotoxic compound is selected from:
- chemotherapy agents such as: cyclophosphamide, busulfan, melphalan, ifosfamide, bendamustine, temozolomide, bleomycin, mechlorethamine, carmustine, fotemustine, BCNU, cisplatin, oxaliplatin, carboplatin, aracytin, fludarabine, cladribine, clofarabine, gemcitabine, 5 fluorouracil, capecitabine, methotrexate, pemetrexed, 6 mercaptopurine decitabine, azacytidine, troxacitabine, vincristine, vinblastine, vindesine, vinorelbine, vinflunine, paclitaxel, docetaxel, cabazitaxel, epothilones, eribulin, micronized paclitaxel, etoposide, teniposide, irinotecan, camptothecin, doxorubicin, daunorubicin, idarubicin, mitoxantrone, dactinomycin;
- targeted therapy agents of the kinase inhibitor type such as: imatinib, dasatinib, bosutinib, nilotinib, ponatinib, ibrutinib, idelalisib, afatinib, erlotinib, gefitinib, lapatinib, crizotinib, ruxolitinib, tofacitinib, axitinib, cabozantinib, nindetanib, pazopanib, vandetanib, regorafenib, sorafenib, sunitinib, dabrafenib, trametinib, vemurafenib;
- immunomodulators such as thalidomide, lenalidomide, pomalidomide;
- apoptosis promoters such as venetoclax or navitoclax;
- other antitumor agents such as bortezomib, carfilzomib, ixazomib, vorinostat, panobinostat, romidepsin, all-trans retinoic acid, arsenic derivative As₂O₃, temsirolimus, olaparib, rucaparib, pentostatin, asparaginase, hydroxyurea, and chloraminophene.

8. The pharmaceutical combination product as claimed in claim 7, **characterized in that** the cytotoxic compound is a chemotherapy agent.

9. The pharmaceutical combination product as claimed in one of claims 1 to 8, for anti-tumor therapeutic use.

10. The pharmaceutical combination product for use as claimed in claim 9, **characterized in that** the tumor is a liquid tumor or a solid tumor.

11. The pharmaceutical combination product for use as claimed in one of claims 9 or 10, **characterized in that** the tumor is selected from the group consisting of: a breast tumor, a lung tumor, a skin tumor (melanoma), a blood tumor (leukemia), a bone tumor and a lymphoma.

12. The pharmaceutical combination product for use as claimed in one of claims 9 to 11, **characterized in that** the use of each component is simultaneous, separate or sequential.

13. The pharmaceutical combination product for use as claimed in one of claims 9 to 12, **characterized in that** the liposomal formulation is adapted for systemic administration.
